# EUROPÄISCHE PATENTANMELDUNG

(11) **EP 0 994 348 A2**
(43) Veröffentlichungstag der Anmeldung: **19.04.2000**
(21) Anmeldenummer: 99117768.4
(22) Anmeldetag: 09.09.1999
(51) Int. Cl.: G01N 27/49

(54) **Verfahren und Vorrichtung zur Bestimmung Von O2 und N2O in Gasgemischen**

(30) Priorität: 16.10.1998 DE 19847707
(71) Anmelder: VARTA Gerätebatterie GmbH, 30419 Hannover (DE)
(72) Erfinder: Holl, Konrad, Dr., 73434 Aalen-Dewangen (DE); Ilic, Dejan, Dr., 73479 Ellwangen (DE); Schmalz, Michael, Dr., 73479 Ellwangen-Eggenrot (DE); Kohnke, Hans-Joachim, Dr., 34123 Kassel (DE)
(74) Vertreter: Kaiser, Dieter Ralf, Dipl.-Ing.

(57) **Zusammenfassung**

Die Erfindung betrifft ein Verfahren zur Bestimmung von O₂ und N₂O in Gasgemischen durch quantitative elektrochemische Umsetzung, bei dem einer Zink-Luft-Zelle ein Gasgemisch zugeführt wird, wobei die Gaskathode mit dem Gasgemisch in Verbindung steht und die Elektroden der Zink-Luft-Zelle an ein Netzwerk, bestehend aus Dioden und Widerständen angeschlossen sind und auf mindestens zwei elektrochemische Arbeitsbereiche eingestellt werden, wobei der im jeweiligen Arbeitsbereich fließende Strom zur Anzeige des Anteils von O₂ und N₂O im Gasgemisch verwendet wird.

Die Erfindung betrifft weiterhin eine Vorrichtung zur Durchführung des Verfahrens, welche aus einem das Gasgemisch (5) und eine Zink-Luft-Zelle (1) aufnehmenden Gehäuse (2,3) und einer mit den Elektroden der Zink-Luft-Zelle (1) verbundenen Schaltung besteht, die Dioden (7,8) und Widerstände (9,10) sowie Abgriffspunkte (11,12) und Anordnungen zur Messung und Anzeige der Stromstärke enthält.

## Beschreibung

Die Erfindung betrifft ein Verfahren und eine Vorrichtung zur Bestimmung von Sauerstoff (O₂) und Lachgas (N₂O) in Gasgemischen durch quantitative elektrochemische Umsetzung.

Aus dem Dokument US-Re 31,914 ist ein elektrochemisches Verfahren zur Bestimmung von giftigen Gasen in der Luft bekannt. Weiterhin beschreiben die Dokumente US-A 4,495,051, US-A 4,894,138 und US-A 4,914,424 galvanische Zellen, die als Sauerstoffsensoren eingesetzt werden. Insbesondere offenbart das Dokument WO 97/35186 ein Verfahren und eine Vorrichtung zur Bestimmung des Sauerstoffanteils in der medizinischen Beatmungsgeräten zugeführten Atemluft. Dabei wird eine alkalische Zink-Luft-Zelle als Sensor eingesetzt.

Alkalische Zink-Luft-Zellen werden als Knopfzellen in großen Stückzahlen weltweit hergestellt und als Primärzellen besonders in Hörgeräten verwendet. Begrenzt man die Heranführung des Sauerstoffs durch Strömungs- und/oder Diffusionshemmnisse, so wird der maximal lieferbare Strom zum Grenzstrom und ist dann nur noch von der Sauerstoffkonzentration des an die O₂-Kathode angrenzenden Mediums abhängig. Die Zink-Luft-Zelle wird damit zu einer Sensorzelle für die Sauerstoffkonzentrationsmessung oder für die Sauerstoffmengenmessung beim Einsatz in einem abgeschlossenen Volumen.

Bei der Überwachung der Atemluft von Patienten in der Lachgas-Narkose wurde gefunden, daß die Messung des Sauerstoffgehaltes allein nicht ausreichend ist, sondern daß auch die Lachgaskonzentration kontrolliert werden muß.

Die Erfindung hat sich die Aufgabe gestellt, ein Verfahren und eine Vorrichtung zur Bestimmung von Sauerstoff (O₂) und Lachgas (N₂O) in Gasgemischen anzugeben, mit der Sauerstoff und Lachgas in der Atemluft nebeneinander bestimmt werden können.

Die Aufgabe wird erfindungsgemäß durch das gattungsgemäß in Anspruch 1 angegebene Verfahren mit den dort dargelegten Merkmalen gelöst. Weitere vorteilhafte Ausgestaltungen des Verfahrens, die erfindungsgemäße Vorrichtung sowie vorteilhafte Ausgestaltungen der Vorrichtung sind in den Unteransprüchen angegeben.

Die Einstellung der elektrochemischen Arbeitsbereiche wird durch die Einstellung von Spannungsbereichen vorgenommen. Dabei wird ein unterer Spannungsbereich von 0,4 bis 0,8 V und ein oberer Spannungsbereich von 0,8 bis 1,5 V eingestellt.

Die nahezu gleichzeitige Bestimmung von Lachgas und Sauerstoff erfolgt vorzugsweise dadurch, daß zur Einstellung des unteren Spannungsbereichs eine Diode zeitweilig überbrückt wird.

Die erfindungsgemäße Vorrichtung zur Durchführung des Verfahrens besteht aus einem das Gasgemisch und eine Zink-Luft-Zelle aufnehmenden Gehäuse, einer mit den Elektroden der Zink-Luft-Zelle verbundenen Schaltung, welche Dioden und Widerstände, sowie Abgriffspunkte und Anordnungen zur Messung und Anzeige der Stromstärke enthält. Die Schaltung enthält vorzugsweise einen periodisch betätigbaren Überbrückungsschalter, der mindestens einer der Dioden parallel geschaltet ist. Die Schaltung wird in der Weise weiter ausgebildet, daß die Dioden Zener-Dioden sind.

Umfangreiche experimentelle Untersuchungen haben ergeben, daß sich an der Sauerstoffelektrode einer Zink-Luft-Zelle mit Aktivkohle als Katalysator Lachgas inert verhält. Nur an Elektroden aus Silber oder mit Silber katalysierter Kohle sowie an Elektroden mit Platin als Katalysator findet man im Spannungsbereich zwischen 0,45 V und 0,9 V eine kathodische Reduktion des Lachgases, bei der Stickstoff als Reaktionsprodukt erscheint. In diesem Bereich aber reagiert auch Sauerstoff.

Für die Lösung der Aufgabe, die Konzentration von Sauerstoff und Lachgas in einem Mischgas mit einer Zelle zu bestimmen, wurde die folgende Methode entwickelt. Sie wird vom Spannungsbereich einer Zelle mit einer Zinkanode und einer Gaskathode in alkalischer Lösung bestimmt. Unterhalb einer Spannung von 0,4 V fließt in inerter Atmosphäre ein elektrischer Strom mit kathodischer Wasserstoffentwicklung an der Gaselektrode. Bei Anwesenheit von Lachgas wird dieses kathodisch reduziert, der Stromfluß endet bei etwa 0,9 V. Bei höheren Spannungen ist das Lachgas inert. Bei Anwesenheit von Sauerstoff wird dieser im ganzen Spannungsbereich bis etwa 1,45 V reduziert. Ab 1,6 V ist auch Sauerstoff inert.

Erfindungsgemäß erfolgt die Bestimmung von Sauerstoff und Lachgas nebeneinander in der einer Zelle zugeführten Atemluft dadurch, daß nacheinander zwei Strommessungen in unterschiedlichen Spannungsfenstern durchführt werden. Ein unteres Spannungsfenster erfaßt den Spannungsbereich von 0,4 V bis 0,8 V und ein oberes Spannungsfenster den Spannungsbereich von 0,8 - 1,5 V.

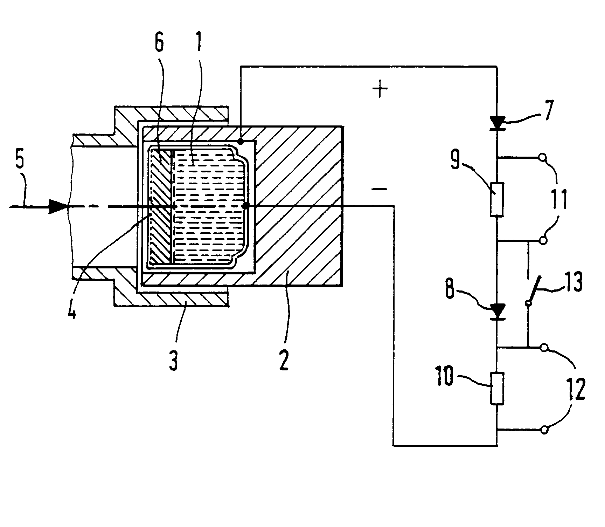

Eine erfindungsgemäße Meßvorrichtung mit einer Schaltung, mit der zwei Spannungsfenster erzeugt werden, ist in Figur 1 schematisch dargestellt.

Gemäß der Figur befindet sich eine handelsübliche Zink-Luft-Knopfzelle 1 in einer Aufnahmehalterung 2. Die Aufnahmehalterung 2 ist in den nur teilweise dargestellten Gasprobenraum 3 so eingesetzt, daß die Gaseintrittsöffnung 4 der Zink-Luft-Knopfzelle 1 zum Gasprobenraum 3 zeigt. Das Gasgemisch 5, welches im wesentlichen Stickstoff, Sauerstoff, Lachgas und Kohlendioxid enthält, steht dadurch in Kontakt zur Gaskathode 6 der Zink-Luft-Knopfzelle 1. Die an die Zink-Luft-Knopfzelle 1 angeschlossene Schaltung besteht aus Dioden 7, 8, vorzugsweise Zener-Dioden und Widerständen 9, 10. Die Dioden 7 und 8 bestimmen ein unteres Spannungsfenster, welches an den Abgriffpunkten 11 ausgewertet wird. Weiterhin ist ein Überbrückungsschalter 13 vorgesehen. Mit dem Überbrückungsschalter 13 kann die Diode 8 überbrückt werden, so daß dann Diode 7 allein ein oberes Spannungsfenster für die Gaszelle bestimmt. Sobald Sauerstoff vorhanden ist, kann in dem unteren Spannungsfenster an den Abgriffpunkten 11 und in dem oberen Spannungsfenster an den Abgriffpunkten 12 ein Strom gemessen werden, der in beiden Spannungsfenstern bei Abwesenheit von Lachgas gleich groß ist, wenn die Grenzstrombedingung erfüllt ist. Ist nur Lachgas vorhanden, so fließt Strom nur im oberen Spannungsfenster, das untere Spannungsfenster bleibt stromlos. Sind Lachgas und Sauerstoff vorhanden, so fließen in den beiden Spannungsfenstern unterschiedliche Ströme, aus denen durch - beispielweise gewichtete - Differenzbildung die Konzentrationen von Sauerstoff und Lachgas im Gasgemisch bestimmt werden können.

Die Betätigung des Schalters 13 ermöglicht den Wechsel von einem Spannungsfenster in das andere. Für die Einstellung des stationären Stromes benötigt die Meßanordnung eine gewisse Zeit, die durch die Zeitkonstanten der mechanischen und elektrochemischen Vorgänge und damit durch die konstruktiven Gegebenheiten bestimmt wird. Wird der Schalter 13 mit einer gewissen Frequenz als Flip-Flop hin- und hergeschaltet, so entsteht ein Stromsignal mit einer Gleichstrom- und einer Wechselstromamplitude. Letztere ist ein Maß für den Lachgasanteil.

## Patentansprüche

1. Verfahren zur Bestimmung von O₂ und N₂O in Gasgemischen durch quantitative elektrochemische Umsetzung, dadurch gekennzeichnet, daß einer Zink-Luft-Zelle ein Gasgemisch zugeführt wird, wobei die Elektroden der Zink-Luft-Zelle an ein Netzwerk, bestehend aus Dioden und Widerständen angeschlossen sind, welches auf mindestens zwei elektrochemische Arbeitsbereiche eingestellt wird und der im jeweiligen Arbeitsbereich fließende Strom zur Anzeige des Anteils von O₂ und N₂O im Gasgemisch verwendet wird.

2. Verfahren nach Anspruch 1, dadurch gekennzeichnet, daß die Einstellung der elektrochemischen Arbeitsbereiche durch die Einstellung von Spannungsbereichen vorgenommen wird.

3. Verfahren nach Anspruch 1 und/oder Anspruch 2, dadurch gekennzeichnet, daß ein unterer Spannungsbereich von 0,4 bis 0,8 V und ein oberer Spannungsbereich von 0,8 bis 1,5 V eingestellt wird.

4. Verfahren nach Anspruch 3, dadurch gekennzeichnet, daß zur Einstellung des oberen Spannungsbereichs eine Zenerdiode zeitweilig überbrückt wird.

5. Vorrichtung zur Durchführung des Verfahrens nach einem oder mehreren der Ansprüche 1 bis 4, bestehend aus einem das Gasgemisch (5) und eine Zink-Luft-Zelle (1) aufnehmenden Gehäuse (2,3), einer mit den Elektroden der Zink-Luft-Zelle (1) verbundenen Schaltung, welche Dioden (7,8) und Widerstände (9,10) sowie Abgriffspunkte (11,12) und Anordnungen zur Messung und Anzeige der Stromstärke enthält.

6. Vorrichtung zur Durchführung des Verfahrens nach Anspruch 5, dadurch gekennzeichnet, daß mindestens einer der Dioden (7,8) ein periodisch betätigbarer Überbrückungsschalter (13) parallel geschaltet ist.

7. Vorrichtung zur Durchführung des Verfahrens nach einem oder mehreren der Ansprüche 4 bis 6. dadurch gekennzeichnet, daß die Dioden (7,8) Zener-Dioden sind.
